# EUROPEAN PATENT SPECIFICATION

(11) **EP 2 810 656 B1**
(45) Date of publication and mention of the grant of the patent: **02.08.2017**
(21) Application number: 13002930.9
(22) Date of filing: 06.06.2013
(51) Int. Cl.: A61K 47/02, A61K 47/12, A61K 31/165

(54) **Agomelatine formulations comprising agomelatine in the form of co-crystals**
Agomelatin-Formulierungen, enthaltend Agomelatin in Co-Kristallform
Formulations de l'agomélatine, comprenant l'agomélatine sous forme de co-cristaux

(43) Date of publication of application: 10.12.2014
(73) Proprietor: Zentiva, k.s., 102 37 Praha 10 (CZ)
(72) Inventor: Rezac, Jaroslav, 277 11 Neratovice (CZ); Pribyl, Daniel, 150 00 Praha 5 (CZ); Hanovska, Anna, 149 00 Praha 11 (CZ); Dammer, Ondrej, 338 44 Dobriv (CZ)
(74) Representative: Jirotkova, Ivana

(56) References cited:
- EP-A1- 1 800 669
- EP-A1- 2 532 647

## Description

### Field of the invention

The objects of this invention are pharmaceutical formulations comprising agomelatine co-crystals with organic acid and manufacturing processes for preparation of such formulations.

### Background of the invention

Agomelatine (N-[2-(7-methoxy-1-naphthalenyl)ethyl]acetamide), its preparation and its pharmaceutical use in treatment of stress, sleep disorders, anxiety seasonal depression, insomnia and tiredness due to "jet lag", schizophrenia, panic attacks, melancholy, the regulation of appetite, insomnia etc. were first described in the priority patent application FR2658818. The effective dose was suggested to vary according to the age and weight of the patient, the route of administration or the indications for treatments within a range of 0.1 mg to 1 g in 24 hours. A tablet consisting of 50 mg agomelatine derivative (N-[2-(7-methoxy-1-naphthalenyl)ethyl]butyramide), wheat starch, corn starch, lactose, magnesium stearate, silica and hydroxypropylcellulose was disclosed.

At present, agomelatine is registered and marketed by Les Laboratoires Servier Company under a trade name Valdoxan® and it is indicated for treatment of major depressive episodes in adults. The recommended dose of agomelatine is 25 mg once daily taken orally and if there is no improvement of symptoms, the dose may be increased to 50 mg once daily, i.e. two 25 mg tablets. The Valdoxan® tablets are film-coaced tablets with tablet cores consisting of agomelatine, lactose monohydrate, maize starch, povidone, sodium carboxymethyl starch, stearic acid, magnesium stearate and anhydrous colloidal silica which are coated by a coat consisting of hypromellose, glycerol, macrogol, magnesium stearate and colourants (yellow ferric oxide, titanium oxide and black ink). The manufacturing process is wet granulation and agomelatine is present in the polymorphic form II in Valdoxan®.

Use of agomelatine in obtaining medicaments intended for the treatment of Generalized Anxiety Disorder was disclosed in patent family of WO2007116136 (Les Laboratoires Servier). The useful dosages were described as varying between 1 mg and 50 mg of agomelatine per day. A pharmaceutical composition was disclosed, consisting of 25 mg agomelatine, lactose monohydrate, magnesium stearate, povidone, anhydrous colloidal silica, cellulose sodium glycolate and stearic acid. A clinical study with a 25 mg agomelatine per day was carried out, and the dose was increased to 50 mg agomelatine per day in cases of low response after treatment for two weeks.

Similarly to above mentioned WO2007116136 (Les Laboratoires Servier), the same pharmaceutical formulation and the same dosing (25 mg, respectively 50 mg per day) were used in a clinical study on the treatment of sleep disorders among depressed patients (WO2007028905 by Les Laboratoires Servier). Furthermore, the same pharmaceutical formulation and dose of 25 mg with possibility of increasing to 50 mg per day was suggested for the treatment of periventricular leukomalacia (WO2008071869 by Les Laboratoires Servier) and for the treatment af Smith-Magenis syndrome (WO2008071870 by Les Laboratoires Servier).

Different types of oral dosage forms were described in the prior art documents. A general controlled release pharmaceutical composition, where agomelatine is mentioned as one of the possible active ingredients, is described in EP1064935 (Les Laboratoires Servier). A solid orodispersible pharmaceutical composition of agomelatine is described in WO03061644 and WO2007068829 (Les Laboratoires Servier). A capsule composition of agomelatine, mannitol, silica and pre-gelatinized starch was disclosed in CN101991559 (Tianjin Hankang Pharmaceutical Biotechnology).

A method for preparation of a solid pharmaceutical formulations by wet granulation is described in WO2011079608 (Shanghai Zhongxi Pharmaceutical Corporation). Agomelatine (25 mg) was dissolved in a acidifier-containing acid solution (ethanol and hydrochloric acid), homogeneously mixed with an alkalizer (natrium hydroxide) and pharmaceutically excipients (povidone). This granulation liquid was used in a fluid spray granulator for granulation of lactose and starch. Carboxymethyl starch sodium and magnesium stearate were admixed after wet granulation and the mixture was compressed into tablet cores which were then film-coated.

There were described many polymorhic forms of agomelatine, e.g. form II (patent family of WO05077887 by Les Laboratoires Servier), form III (patent family of WO2007015003 by Les Laboratoires Servier), form IV (patent family of WO2007015002 by Les Laboratoires Servier), form V (patent family of WO2007015004 by Les Laboratoires Servier), form VI (patent family of WO2009095555 by Les Laboratoires Servier).

As stated above, the polymorphic form II was disclosed in the patent application WO05077887 (Les Laboratoires Servier). In this patent application were described formulations comprising 25 mg agomelatine of polymorphic form II and further consisting of filler (lactose monohydrate, maltodextrin), lubricants (magnesium stearate and stearic acid), binders (pregelatinized starch or povidone), disintegrants (starch or cellulose sodium glycolate), and glidants (anhydrous colloidal silica). The polymorphic form 11 was lately disclosed as a preferred one in all the patent applications on the new medical uses of agomelatine (WO2007116136, WO2007028905, WO2008071869, and WO2008071870, all by Les Laboratoires Servier).

Besides the polymorphic forms of agomelatine, co-crystals of agomelatine were disclosed in the prior art documents. The term "cocrystal" or "co-crystal" is understood to be a binary molecular crystal containing the molecules of the active pharmaceutical ingredient together with another molecular species in a defined stoichiometric ratio where both components are in their neutral state. The terms "cocrystal" and "co-crystal" are generally understood to be synonymous terms referring to such a system. The second component in the co-crystal (the component other than the active pharmaceutical ingredient) is commonly referred to as a "co-crystal former". Generally, pharmaceutically acceptable co-crystal formers include any molecule considered acceptable as a counterion for a pharmaceutical salt or known as a pharmaceutical excipient. A widely accepted definition of a pharmaceutical co-crystal is a crystalline system containing an active pharmaceutical molecule and a co-crystal former that is a solid at ambient temperature and pressure in a defined stoichiometric ratio, although a co-crystal is not limited to containing only two components. The components of the co-crystal are linked by hydrogen bonding and other non-covalent and non-ionic interactions (Aakeroy and Salmon, CrystEngComm, 2005, 439-448). This definition distinguishes co-crystals from crystalline solvates, in which case one of the components is a liquid at ambient temperature and pressure.

Co-crystals of agomelatine were disclosed in patent applications WO2012146371 (Zentiva) and WO2012168665 (Les Laboratoires Servier).

The earlier patent application WO2012146371 (Zentiva) discloses the co-crystals of agomelatine with a co-crystal former possessing at least two carboxylic or at least one sulfonic group in its molecule and methods of preparing these co-crystals which can be used in the preparation of pharmaceutical formulations for the treatment of depression or major depressive disorder. These co-crystals could be prepared reproducibly and had bioavailability, stability, hygroscopicity and mechanical properties that make them suitable for use in the preparation of pharmaceutical formulations that can satisfy the regulations in force governing the quality of pharmaceutical preparations. Co-crystals with citric acid, maleic acid or benzenesulfonic acid and methods of their preparation are specified in the examples.

The later patent application WO2012168665 (Les Laboratoires Servier) discloses co-crystals of agomelatine with organic acids (e.g. with para-hydroxybenzoic acid, citric acid, oxalic acid, gallic acid, maleic acid, malonic acid, glutaric acid, glycolic acid and ketoglutaric acid) and their preparation. The co-crystals were reported to be advantageous for usage in the pharmaceutical formulations because it was possible to modify (promote or reduce) the dissolution rate of agomelatine in comparison to the marketed product Valdoxan®. The co-crystals according to the patent application can be used for preparation of pharmaceutical formulations for oral, parenteral or nasal administration, such as different types of tablets, granules, capsules, lozenges, suppositories, creams, ointments, dermal gels, injections etc. The dosage can, according to the disclosure of the patent application, vary from 0.1 mg to 1 g of agomelatine per day in one or more administrations. An immediate-release tablet is disclosed, consisting of co-crystal of agomelatine with para-hyroxybenzoic acid (corresponding to 25 mg agomelatine), lactose monohydrate, magnesium stearate, maize starch, maltodextrin, anhydrous colloidal silica and pregelatinised maize starch. A retarded-release tablet, consisting of agolematine co-crystal with ketoglutaric acid (corresponding to 25 mg agomelatine), lactose monohydrate, magnesium stearate, povidone, anhydrous colloidal silica and hypromellose, is disclosed as well.

### Summary of the invention

The usage of agomelatine in the form of co-crystals improves the bioavailability of agomelatine. However, the agomelatine co-crystals with organic acid showed to exhibit undesirable technological properties that made the manufacturing processes of pharmaceutical formulations comprising agomelatine co-crystals with organic acid challenging. Special attention had to be paid to the problem of sticking of the mass and the poor flow properties of the mass comprising agomelatine co-crystals.

When solving these problems, following parameters showed to be significant for the effect of the invention:
▪ excluding liquids from the process as well as from the formulation
▪ usage of agomelatine co-crystals in the form of their pre-blend with a glidant
▪ sufficient concentration level of the glidant

When solving these problems, following parameters showed to be insignificant for the effect of the invention:
▪ particle size of the agomelatine co-crystals
▪ type of used dry process
▪ for tablets, the compressing force

Therefore, the object of this invention is a pharmaceutical formulation of agomelatine for oral use comprising
- agomelatine in the form of agomelatine co-crystals with organic acids and
- pharmaceutically acceptable excipients
   characterised in that
- the agomelatine co-crystals are used in the form of pre-blend of the agomelatine co-crystals with a glidant,
- the ratio of the agomelatine co-crystals to the glidant in the pre-blend is lower than or equal to 30:1, and
- the free-water content in the used pharmaceutically acceptable excipients is lower than or equal to 7% w/w.

Another aspect of this invention is a manufacturing process of preparation of pharmaceutical formulation for oral use comprising agomelatine, characterized in that the manufacturing process is a dry process and it comprises pre-blending step of agomelatine in the form of agomelatine co-crystals with organic acids and at least one glidant, wherein the ratio of the agomelatine co-crystals to the glidant in the pre-blend is lower than or equal to 30:1.

### Brief description of the figures

Figure 1: Dissolution profiles of two batches of formulation according to present invention (line 1 and line 3) and two batches of marketed Valdaxan® formulations (line 2 and line 4) at pH 2.0.

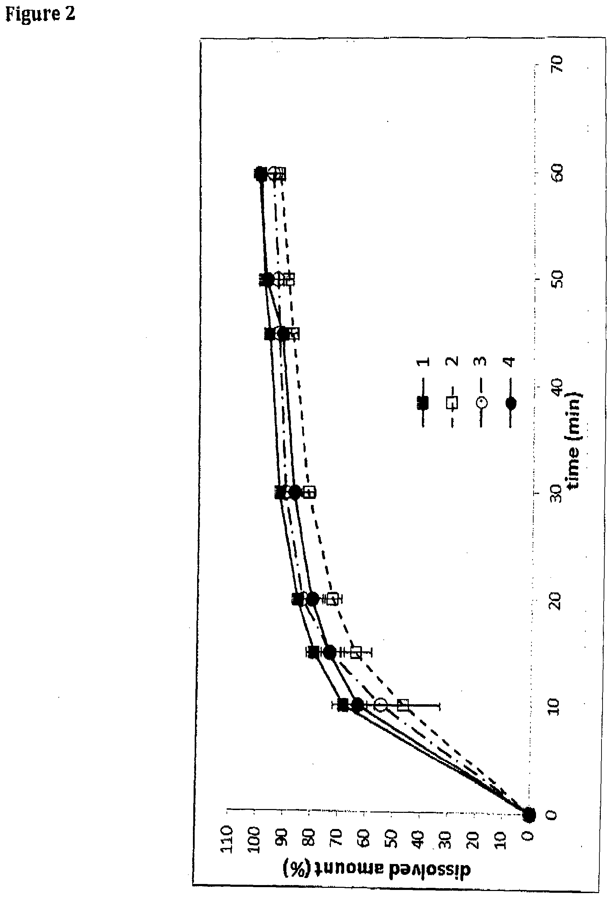

Figure 2: Dissolution profiles of two batches of formulation according to present invention (line 1 and line 4) and two batches of marketed Valdoxan® formulations (line 2 and line 3) at pH 4.5.

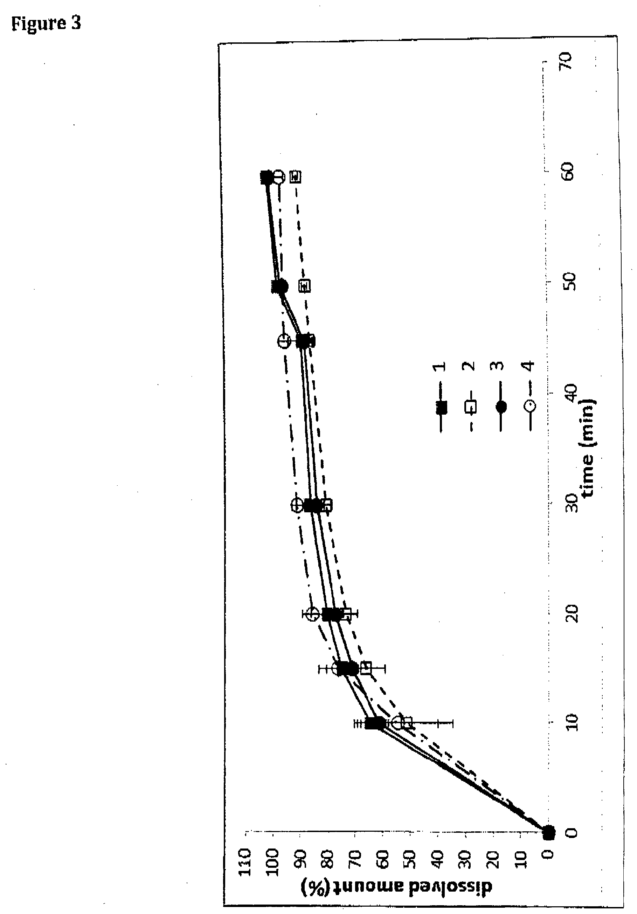

Figure 3: Dissolution profiles of two batches of formulation according to present invention (line 1 and line 3) and two batches of marketed Valdoxan® formulations (line 2 and line 4) at pH 6.8.
Figure 4: Influence of particle size on dissolution profile of agomelatine (line 1- D90 = 130 µm, line 2 - D90 = 12 µm, line 3 - Z90 = 60 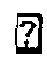 m).

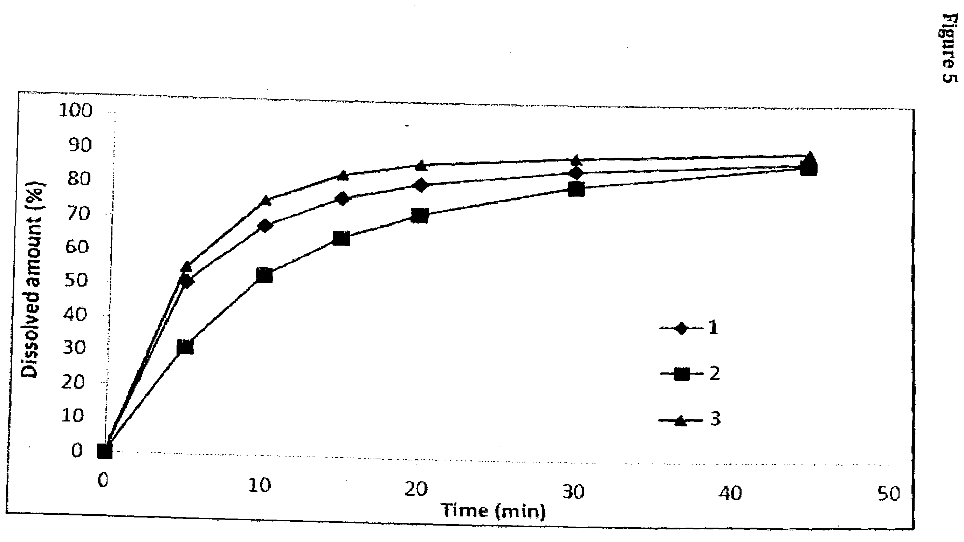

Figure 5: Influence of tablet hardness on dissolution profile of agomelatine (line 1- 96 N, line 2 -146 N, line 3 - 66 N).

### Detailed description

The composition and manufacturing process used for preparation of the original product is not fully applicable for preparation of the product according to the present invention due to different physical properties of the co-crystals. Due to undesirable physical properties of agomelatine co-crystal with organic acids from manufacturing point of view, a special attention had to be paid to overcome problems concerning sticking and poor flow properties of the tableting mixture.

The major problem that had to be overcome was a problem of sticking of the mass to the equipment, e.g. in and to the compactor within granulation process, however, mainly to the surface of the punches during the compression. The flow properties of the tableting mixture and the stability of the composition needed to be improved significantly as well. Moreover, composition and manufacturing process of the present invention product is designed to overcome technology challenges and stability issues of dosage form containing agomelatine.

The first obvious approach for supporting the stability and preventing the decomposition of the co-crystals of agomelatine was usage of dry processes for manufacturing the final pharmaceutical formulation; however, no technological challenges were expected. Surprisingly, immense problems with sticking of the mass together and to the equipment occurred during the common dry processes (i.e. processes where any usage of liquids is excluded - dry granulation, roller compaction, direct compression), and it was not possible even complete the manufacturing process.

This failure was attributed to relatively high water content in some of the used excipients. The process was thus repeated with excipients with free-water content lower than 7 %. However, there was again observed massive sticking of the material during the granulation and mainly during the compression. The problem was of such extend that the usage of agomelatine co-crystals seemed to be inapplicable for industrial preparation of pharmaceutical formulations in this phase of development.

Further attempts were carried out though, and after many experiments parameters were found that enable not only the laboratory but as well the industrial scale production of pharmaceutical formulations comprising agomelatine co-crystals. The most substantial tests that leaded to the present invention are described herein below and are illustrated by the Examples.

The object of this invention is therefore a pharmaceutical formulation comprising agomelatine co-crystal and a manufacturing process suitable for its industrial scale production.

Following features proved to be substantial for the technical effect of this invention:
- the water content in the pharmaceutical formulation
- the agomelatine co-crystal pre-blend with glidant
- the glidant amount

Features that showed not to be crucial for the technical effect of this invention are:
- the particle size of the co-crystals
- the type of dry process
- the tablet hardness (if the formulation is in the form of tablets)

### The water content in the pharmaceutical formulation

The water content has to be minimalized in the pharmaceutical formulation. This can be obviously achieved by excluding wet processes and by usage of pharmaceutically acceptable excipients that have low free-water content and that are non-hygroscopic. The low free-water content is defined as lower than or equal to 7% w/w.

Excluding of the humidity from the manufacturing process and from the pharmaceutical formulation is considered to be an obvious but essential condition for achieving the effect of the invention.

### The agomelatine co-crystal pre-blend with glidant

In the common pharmaceutical praxis, glidants are usually used in tablet manufacturing processes and the glidants are added to the mixture just prior to compression in to the tablets in order to improve the flow properties of the material. In the common processes, the glidants are used in the amount up to 10% w/w.

However, when preparing the pharmaceutical formulation of agomelatine co-crystal and adding the glidant routinely at the last step prior compression, major sticking of the material to the punches occurred during the compression. The addition of the glidant just prior compression of the tablet mass was by no means sufficient to avoid the problem with the material sticking, even when the amount of glidants was increased significantly above the glidant levels commonly used.

Surprisingly, it was found that the problem of sticking was overcome when part of the glidant was used in the form of its pre-blend with agomelatine co-crystal in the pharmaceutical formulation, and even the commonly used glidant concentration levels (up to 10% w/w of the total formulation) were sufficient.

It is to be noted that not only the tablet pharmaceutical forms are the object of this invention. It applies for any other oral pharmaceutical dosage form (e.g. capsules, granules in sachets etc.) that the sticking problem is solved by usage of the agomelatine co-crystal in the form of its pre-blend with a glidant.

### The glidant amount

The usage of the pre-blend of agomelatine co-crystal with the glidant itself is not sufficient for achieving the effect of the present invention. As it is shown by the examples, not only the usage of the pre-blend but the sufficient concentration of glidant as well is fundamental to achieve the effect of the invention, i.e. prepare formulation comprising co-crystal.

In other words, pre-blending of agomelatine co-crystal with the glidant is not sufficient for solving the problem with sticking of the mass, if the used concentration of the glidant is not high enough. However, this applies vice versa as well - the sufficiently high concentration of glidant does not solve the technological problem with the sticking of the mass by itself without carrying out the pre-blending step.

The total glidant amount in the final pharmaceutical formulation thus lies within 2 and 10% w/w; however, at least part of the total amount (e.g. 1.7-9.7% w/w for tablets or 1.7-10% w/w for capsules) must be used in the form of a pre-blend with the agomelatine co-crystal to achieve the technical effect of this invention.

The ratio of the agomelatine co-crystal to the glidant in the pre-blend must not exceed the value of 30:1 to achieve the technical effect of this invention. Typically, the ratio of the agomelatine co-crystal to the glidant in the pre-blend is lower than 15:1 and most preferably it is between 5:1 and 10:1.

### The particle size of the co-crystals

The effect of the particle size of the agomelatine co-crystals on the sticking of the mass within the production of pharmaceutical formulations comprising agomelatine co-crystals was evaluated; however, no such effect was observed.

Agomelatine co-crystals with particle size distribution characterized by D90 values of 12 µm, 60 µm, 130 µm and 200 µm were used for preparation of the pharmaceutical formulations. In all cases, if the agomelatine co-crystals were used in the form of the pre-blend with the glidant and the glidant amount was sufficient as defined above, no technological problems occurred. On the other hand, in all cases, if the agomelatine co-crystals were not pre-blended with the glidant, massive sticking of the mass occurred and it was impossible to complete the manufacturing process.

The effect of the particle size of the agomelatine co-crystals on the dissolution profiles was evaluated as well and no significant influence was observed.

Thus, the agomelatine co-crystals of the particle size distribution characterized by D90 values within the range of 10 - 200 µm are suitable for preparation of pharmaceutical formulations comprising agomelatine in the form of co-crystals.

### The type of dry process

As it was already stated, the water content in the pharmaceutical formulations comprising agomelatine co-crystals has to be minimalized. This can be obviously achieved by excluding wet processes.

For preparation of the pharmaceutical formulations comprising agomelatine co-crystals are suitable dry processes, such as direct compression for preparation of tablets or dry granulation, especially roller compaction, for preparation of granules. The granules comprising agomelatine co-crystals can be then optionally mixed with further pharmaceutically acceptable excipients and compressed into tablets or filled into capsules. In each case, however, the agomelatine co-crystals have to be pre-blended with a glidant in maximal ratio of the agomelatine co-crystals to the glidant being 30:1 prior to the direct compression or prior to the dry granulation.

When the dry compression and the dry granulation by roller compaction was carried out without pre-blending of the agomelatine co-crystals with the glidant, massive sticking of the mass to the equipment occurred during the production process.

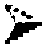 *dry granulation*

Dry granulation process is suitable for medium- and high-dose drugs and is particularly applicable for active pharmaceutical ingredients that are moisture sensitive. This technology is more effective because of process expedition, easy handling, and time and cost savings. The dry granulation process can be described as follows:

### A/ PRE-BLENDING

Agomelatine co-crystals and a glidant are sieved and blended in a container. The maximal ratio of the agomelatine co-crystal to the glidant is 30:1 w/w, typically, the ratio is lower than 15:1 and most preferably it is between 5:1 and 10:1. The parameters of blending are common, e.g. blending at 25 - 40 RPM for 5-15 min in Turbula mixer. Optionally, a part of filler (typically one third of the total filler amount) may be used in the pre-blending step as well due to improvement of the flow properties of the glidant. However, the usage of the filler in the pre-blend is not fundamental for achievement of the technical effect of this invention.

### B/ HOMOGENIZATION PRIOR TO DRY COMPACTION

After this pre-blending step other pharmaceutically acceptable excipients may be added into the container according to the common knowledge of any skilled person; e.g. filler(s), binder(s), disintegrant(s) and/or lubricant(s), and blended with the pre-blend formed in the step A/.

### C/ DRY GRANULATION AND SIEVING OF GRANULES

The blend of the step B/ is dry-granulated, preferably by roller compactor, under commonly used conditions and sieved.

### D/ FURTHER PROCESSING OF THE GRANULES

The granules obtained in the step C/ can be compressed into tablets with tablet hardness of 60 - 140 N (according to method described in European Pharmacopeia) or filled into capsules. Prior to either of these options, other excipients (e.g. glidant(s) and/or lubricant(s)) may be added to the granules obtained in the step C/ and homogenized according to the common knowledge of any skilled person. If the granulate is compressed into tablets, the tablets can optionally coated by one or more coatings, e.g. a common film-coat, moisture-protecting coat or by a release-modifying coating.

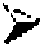 *direct compression*

The process of dry compression can be used for preparation of pharmaceutical formulations in the form of tablets comprising agomelatine co-crystals. The process can be described as follows:

### A/ PRE-BLENDING

Agomelatine co-crystals and a glidant are sieved and blended in a container. The maximal ratio of the agomelatine co-crystal to the glidant is 30:1 w/w, typically, the ratio is lower than 15:1 and most preferably it is between 5:1 and 10:1. The parameters of blending are common, e.g. blending at 25 - 40 RPM for 5-15 min in Turbula mixer. Optionally, a part of filler (typically one third of the total filler amount) may be used in the pre-blending step as well due to improvement of the flow properties of the glidant. However, the usage of the filler in the pre-blend is not fundamental for achievement of the technical effect of this invention.

### B/ HOMOGENIZATION PRIOR TO THE COMPRESSION

After this pre-blending step other pharmaceutically acceptable excipients may be added into the container according to the common knowledge of any skilled person; e.g. filler(s), binder(s), disintegrant(s) and/or lubricant(s), and blended with the pre-blend formed in the step A/.

### C/ COMPRESSION INTO TABLETS

The blend obtained in the step B/ is compressed into tablets with tablet hardness of 60 - 140 N (according to method described in European Pharmacopeia). Prior to compression other excipients (e.g. glidant(s) and/or lubricant(s)) may be added and homogenized with the granules obtained in the step B/ according to the common knowledge of any skilled person. The tablets can optionally coated by one or more coatings, e.g. a common film-coat, moisture-protecting coat or by a release-modifying coating.

### The tablet hardness

For the pharmaceutical formulations in the form of tablets, no effect of the compressing force on the problem with sticking was observed and no significant influence on dissolution profile was found. Tablet hardness of 60 - 140 N (according to method described in European Pharmacopeia) is suitable for the tablets comprising agomelatine co-crystals.

### Composition of a pharmaceutical formulation according to the invention

The composition of a pharmaceutical formulation according to the invention is shown in the Table 1.

**Table 1: General composition comprising agomelatine in the form of co-crystals**

| Substance/Excipient type | Amount range in the formulation (weight % in the composition) |
|---|---|
| Agomelatine co-crystal* | 10 - 50 |
| Glidants (total amount) | 2 - 10 |
| Glidants*,** | 1.7 - 10 |
| Fillers | 30 - 80 |
| Binders | 1 - 5 |
| Disintegrants | 2 - 10 |
| Lubricants | 0.5 - 7 |
| Film-forming agents | up to 5 |
| Plasticizers | up to 2 |
| Pigments | up to 1 |

| | |
|---|---|
| *in the form of pre-blend **part of the glidant of the total amount of the glidant that is used in the pre-blend with agomelatine co-crystals | |

As said above, suitable pharmaceutically acceptable excipients have free-water content lower than or equal to 7% w/w and are non-hygroscopic. Examples of suitable excipients herein below illustrate the invention but do not limit it:
Fillers: Microcrystalline cellulose, silicified microcrystalline cellulose, anhydrous beta-lactose, alpha-lactose monohydrate, sorbitol powder, mannitol powder, maltitol powder, xylitol powder, calcium phosphate, sucrose, calcium carbonate and any combination thereof.
**Binders:** Polyvinylpyrrolidones, copovidone, hypromellose, hyprollose or other cellulose esters, cellulose ethers and any combination threof.
**Disintegrants:** Croscarmellose sodium (sodium starch glycolate), crospovidone, hydroxypropyl cellulose low-substituted and any combination threof.
**Glidants:** Colloidal anhydrous silica, talc, magnesium carbonate, stearic acid or its metallic salts such as magnesium stearate, sodium stearyl fumarate, magnesium palmitate, magnesium oleate, hydrogenated vegetable oil, hydrogenated castor oil, talc, macrogols (polyethylene glycoles) of different molecular weights and any combination threof.
**Lubricants:** Sodium stearyl fumarate, stearic acid, stearic acid salts such as magnesium stearate, calcium stearate, magnesium palmitat, magnesium oleate, hydrogenated vegetable oil, hydrogenated castor oil, talc, colloidal anhydrous silica, macrogols (polyethylene glycoles), and any mixtures thereof.
**Film-forming agents:** Hypromellose, hyprollose, shellac
**Plasticizers:** Macrogols, glycerol
**Pigments:** Ferric oxides (yellow, red, black), titanium dioxide, riboflavin, quinoline yellow, sunset yellow

The formulations may voluntary comprise surfactants, e.g. sodium lauryl sulfate, polyoxyethylene sorbitan fatty acid esters, polyoxyethylene alkyl ethers, sorbitan fatty acid esters, polyethylene glycols, sugar esters of fatty acids and glycerides of fatty acids or mixtures thereof.

### Definitions

The term "agomelatine co-crystal" is meant as agomelatine co-crystal with organic acid.

The term "cocrystal" or "co-crystal" is understood to be a binary molecular crystal containing the molecules of the active pharmaceutical ingredient together with another molecular species in a defined stoichiometric ratio where both components are in their neutral state. The terms "cocrystal" and "co-crystal" are generally understood to be synonymous terms referring to such a system. The second component in the co-crystal (the component other than the active pharmaceutical ingredient) is commonly referred to as a "co-crystal former". A widely accepted definition of a pharmaceutical co-crystal is a crystalline system containing an active pharmaceutical molecule and a co-crystal former that is a solid at ambient temperature and pressure in a defined stoichiometric ratio, although a co-crystal is not limited to containing only two components. The components of the co-crystal are linked by hydrogen bonding and other non-covalent and non-ionic interactions (Aakeroy and Salmon, CrystEngComm, 2005, 439-448). This definition distinguishes co-crystals from crystalline solvates, in which case one of the components is a liquid at ambient temperature and pressure.

The preferred co-crystal formers of the present invention are citric acid, maleic acid or benzenesulfonic acid.

Still more preferred co-crystal former is citric acid. The preferred molar ratio of agomelatine to the co-crystal former citric acid is 1:1. The term "dry process" means a manufacturing process wherein any usage of liquids is avoided and only pharmaceutically acceptable excipients having low free-water content are used. The "low free-water content" is defined as being lower than or equal to 7% by weight of the respective excipient.

### Examples

Following Examples 1- 5 depict the present invention.

The Examples 1 and 6 illustrate the optimized composition of the pharmaceutical formulation according to the invention. The Example 2 - 5 are comparative examples and were designed in a sequence to demonstrate the effect of the invention.

Example 2 shows the effect of the glidant concentration in the formulation.

Example 3 shows the effect of presence of the agomelatine co-crystal pre-blend with the glidant in the formulation.

In Examples 4 and 5 the effect of agomelatine co-crystal particle size, respectively the tablet hardness on the dissolution profiles was tested.

The dissolution method used for determination of the dissolution profiles can be in all cases described as follows: 900 ml of dissolution medium, paddles, 50 rpm for 45 minutes then 150 rpm, pH of the dissolution media: pH 2 (0.01 M HCl), pH 4.5 (phosphate buffer), pH 6.8 (phosphate buffer).

### Example 1a - A pharmaceutical formulation according to the invention

**Table 2**

| **Ingredient** | **Function** | **Amount in tbl/cps** | |
|---|---|---|---|
| | | mg | % |
| **Tablet core or capsule fill** | | | |
| Agomelatine* | active ingredient | 25.00 | 18.7 |
| Citric acid* | co-crystal former | 19.74 | 14.7 |
| Silicified microcrystalline cellulose | filler, glidant | 60.77 | 45.3 |
| Polyvinylpyrrolidone 30 | binder | 4.06 | 3.0 |
| Colloidal silica | glidant | 6.50 | 4.9 |
| Crospovidone | disintegrant | 7.80 | 5.8 |
| Sodium stearyl fumarate | lubricant | 3.70 | 2.8 |
| Magnesium stearate | lubricant | 0.50 | 0.4 |
| Stearic acid | lubricants | 2.00 | 1.5 |

| **Optionally: film-coating** | | | |
|---|---|---|---|
| Methocel E5 | film-forming agent | 2.39 | 1.8 |
| Macrogol 6000 | plasticizer | 0.57 | 0.4 |
| Titanium dioxide | colourant | 0.30 | 0.2 |
| Talc | colourant | 0.61 | 0.5 |
| Ferric oxide yellow | colourant | 0.12 | 0.1 |
| **Total tablet mass** | | **134.00** | **100.0** |

| | | | |
|---|---|---|---|
| *present together as a parts of co-crystal in the drug product | | | |

### Manufacturing Process:

Two batches of the composition shown in the Table 2 were produced by roller compaction - batch number 420412 and batch number 541012. The granules of the batch number 541012 and part of the granules of the batch number 420412 were compressed into tablets. The remaining granules of the batch number 420412 were filled into capsules.

### A/ PRE-BLENDING

Agomelatine co-crystal with citric acid, one third (by weight) of the total amount of the silicified microcrystalline cellulose and a part of the colloidal anhydrous silica (app. 85% of total amount by weight) were sieved through 0.8 ± 0.2 mm sieve and homogenized by blending in Turbula mixer at 32 ± 2 RPM for 10 min.

### B/ HOMOGENIZATION PRIOR TO DRY COMPACTION

The rest of the silicified microcrystalline cellulose, povidone 30, sodium stearyl fumarate and a part of crospovidon (app. 50 % of total amount by weight) were sieved through 0.8 ± 0.2 mm sieve and added to the pre-blend formed in the step A/ and homogenized again in Turbula mixer at 32 ± 2 RPM for 15 min.

### C/ DRY GRANULATION AND SIEVING OF GRANULES

The homogenized mixture was granulated by roller compactor under pressure 40 - 65 bar and the granulate was sieved through 1.0 ± 0.2 mm sieve.

### D/ PREPARATION OF MIXTURE FOR TABLETING OR FILLING INTO CAPSULES

The rest of the colloidal anhydrous silica and the rest of crospovidon were sieved through 0.8 ± 0.2 mm sieve and added to the granulate of the step C/, and homogenized in Turbula mixer at 32 ± 2 RPM for 20 min. After that stearic acid and magnesium stearate were sieved through the same sieve and added into mixture and homogenized in Turbula mixer at 32 ± 2 RPM for 5 min.

### E1/ COMPRESSION

The mixture ready for compression from the previous step was compressed using a rotary tableting machine at commonly used conditions.

The resulting tablet cores were oblong, biconvex, size of approximately 9 x 4.5 mm (length x width) and average mass 130 mg ± 5 %.

### F1/ COATING

Coating suspension was prepared using Methocel E5, Macrogol 6000, Titanium dioxide, Talc, Ferric oxide yellow and purified water. Cores were film coated using coating suspension in a coater machine. The film-coated tablets were yellow oblong biconvex film-coated tablets, size of approximately 9 x 4.5 mm (length x width) and average mass 134 mg ± 5%.

### E2/ FILLING INTO CAPSULES

The mixture from the previous step was filled into capsules of capsule size 2 with capsule fill 130 mg± 5 %.

### Results:

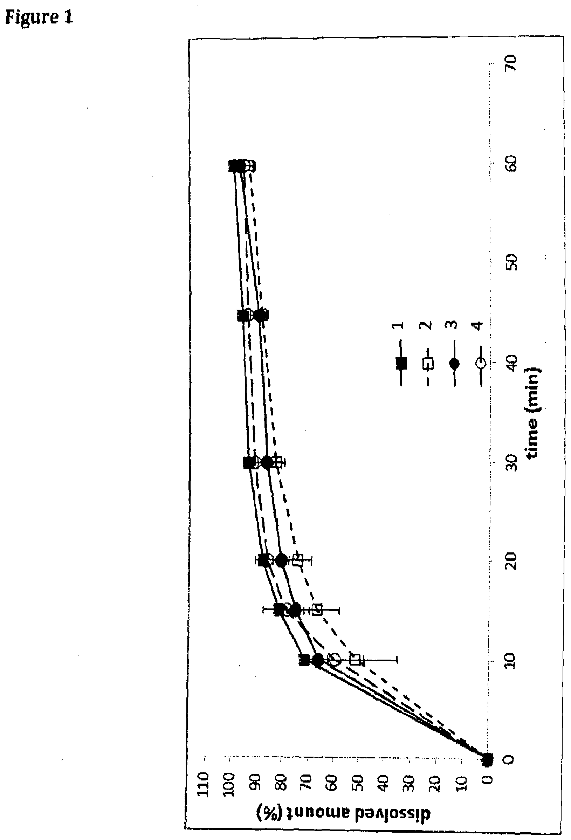

Example 1 corresponds to product batch number 420412 and 541012. These experiments were successful. Manufacturing process was not demanding, no sticking occurred and flow properties were complying. The *in-vitro* dissolution profiles (shown in the figure 1) as well as other physico-chemical parameters (shown in example 1b and in tables 3 and 4) of the product were complying.

### Example 1b - Stability testing of the pharmaceutical formulation according to the invention

Stability tests of Agomelatine tablets (batch number 420412) are shown in the tables 3 and 4. The tablets were stable at storage conditions 40°C / 75 % relative humidity as well as 25°C / 60% relative humidity. The marketed product Valdoxan® was tested at storage conditions 40°C / 75% relative humidity for comparison (table 5). No significant difference between the two products was seen.

**Table 3: Stability of tablets comprising agomelatine co-crystals (batch number 420412) at storage conditions 40°C/75 % relative humidity**

| Test | | Limit | At release | 3 month |
|---|---|---|---|---|
| Appearance | | Yellow oblong biconvex film-coated tablet | Complies | Complies |
| Average mass of 1 film-coated tablet | | 0.127 - 0.141g | 0.136 g | 0.137 g |
| Polymorphism | | Agreement with reference pattern of co-crystal | Complies | Complies |
| Assay of agomelatine in 1 film-coated tablet | | 23.7 - 26.3 mg | 25.3 mg | 25.0 mg |
| Dissolution test | | Q=75 % of declared amount in 30 min | 0̸ 91.1 % | 0̸ 87.8 % |

| Purity tests : | | | | |
|---|---|---|---|---|
| - | Known impurities individually | NMT 0.4 % | < 0.05 % | < 0.05 % |
| - | Unknown impurities individually | NMT 0.2 % | < 0.05 % | < 0.05 % |
| - | Total impurities | NMT 1.0 % | < 0.05 % | < 0.05 % |
| Water | | NMT 6.0 % | 2.1 % | 2.2 % |

**Table 4: Stability of tablets comprising agomelatine co-crystals (batch number 420412) at storage conditions 25°C/60% relative humidity**

| Test | | Limit | At release | 3 month |
|---|---|---|---|---|
| Appearance | | Yellow oblong biconvex film-coated tablet | Complies | Complies |
| Average mass of 1 film-coated tablet | | 0.127 - 0.141 g | 0.136 g | 0.136 g |
| Polymorphism | | Agreement with reference pattern of co-crystal | Complies | Complies |
| Assay of agomelatine in 1 film-coated tablet | | 23.7 - 26.3 mg | 25.3 mg | 24.9 mg |
| Dissolution test | | Q=75 % of declared amount in 30 min | 0̸ 91.1% | 0̸ 85.0 % |
| Purity tests : | | | | |
| - | Known impurities individually | NMT 0.4 % | < 0.05 % | < 0.05 % |
| - | Unknown impurities | NMT 0.2 % | < 0.05 % | < 0.05 % |
| | individually | NMT 1.0 % | < 0.05 % | < 0.05 % |
| - | Total impurities | | | |
| Water | | NMT 6.0 % | 2.1 % | 2.4 % |

**Table 5: Stability of marketed Valdoxan® tablets at storage conditions 40°C/75% relative humidity**

| Test | | Limit | At the beginning of the stability test | 3 month |
|---|---|---|---|---|
| Appearance | | Yellow oblong biconvex film-coated tablet | Complies | Complies |
| Polymorphism | | Agreement with reference pattern | Form II | Form II |
| Assay of agomelatine in 1 film-coated tablet | | 23.7 - 26.3 mg | 24.44 mg | 24.47 mg |
| Dissolution test | | Q=75 % of declared amount in 30 min | 0̸ 91.2 % | 0̸ 90.3 % |
| Purity tests: | | | | |
| - | Total impurities | NMT 1.0 % | < 0.05 % | < 0.05 % |
| Water | | NMT 6.0 % | 4.9 % | 5.0 % |

### Example 2 - Influence of a lower glidant amount on feasibility of the manufacturing process of the tablets comprising agomelatine co-crystals

**Table 6**

| **Agomelatine Film-coated tablet composition** | | | |
|---|---|---|---|
| **Ingredient** | **Function** | **Amount in tablet** | |
| | | mg | % |
| **Tablet core** | | | |
| Agomelatine* | active ingredient | 25.00 | 18.7 |
| Citric acid* | co-crystal former | 19.74 | 14.7 |
| Silicified microcrystalline cellulose | filler, glidant | 66.55 | 49.7 |
| Povidone | binder | 4.06 | 3.0 |
| Colloidal silica | glidant | 0.66 | 0.5 |
| Crospovidone | disintegrant | 7.80 | 5.8 |
| Sodium stearyl fumarate | lubricant | 3.70 | 2.8 |
| Magnesium stearate | lubricant | 0.50 | 0.4 |
| Stearic acid | lubricant | 2.00 | 1.5 |

| **Film-coating** | | | |
|---|---|---|---|
| Methocel E5 | film-forming agent | 2.39 | 1.8 |
| Macrogol 6000 | plasticizer | 0.57 | 0.4 |
| Titanium dioxide | colourant | 0.30 | 0.2 |
| Talc | colourant | 0.61 | 0.5 |
| Ferric oxide yellow | colourant | 0.12 | 0.1 |
| **Total tablet mass** | | **134.00** | **100.0** |

| | | | |
|---|---|---|---|
| *present as a part of co-crystal in the drug product | | | |

### Manufacturing Process:

The manufacturing process of the tablets was identical to that described in the Example 1. The only difference compared to Example 1 is reduced amount of glidant colloidal silica to 0.5 %. The amount of filler was increased adequately to compensate the decrease in the glidant concentration.

### Results:

Despite using the manufacturing process comprising the pre-blending step, sticking of the material to the surface of the punches during compression was not acceptable. The amount of glidant was not sufficient to avoid the sticking, therefore this composition was not suitable for a routine manufacture and the manufacturing process had to be stopped before the coating step.

### Example 3a - Influence of modified manufacturing process without pre-blending of co-crystal, part of filler and silica

This example illustrates the common dry granulation process where glidants as well as lubricants are added just prior to compression to improve flow properties of the material. Other starting materials are homogenized in one single step prior dry granulation. The same composition of ingredients as shown in Example 1 was used for preparing the tablet cores.

### Manufacturing process

### A+B/ HOMOGENIZATION PRIOR TO DRY COMPACTION

Agomelatine co-crystal with citric acid, silicified microcrystalline cellulose, povidone 30, sodium stearyl fumarate and crospovidon were sieved through 0.8 ± 0.2 mm sieve and homogenized in Turbula mixer at 32 ± 2 RPM for 10 min.

### C/ DRY GRANULATION AND SIEVING OF GRANULES

The homogenized mixture prepared in the step A+B is granulated as described in Example 1.

### D/ PREPARATION OF MIXTURE FOR TABLETING

The granulate prepared in the step C was mixed with the colloidal silica and it was further processed as in the Example 1.

### E/ COMPRESSION, F/ COATING

The compression was carried out as described in the Example 1; however, the process had to be stopped due to massive sticking of the mass to the equipment.

### Results:

Although the same qualitative as well as quantitative composition of ingredients as in the Example 1 was used, significant sticking of the material to the equipment, especially to the surface of the punches occurred due to the missing pre-blending step. This manufacturing process is therefore not suitable for a routine manufacture even if the composition is the same as in Example 1.

### Example 3b - Influence of pre-blending on direct compression

The same composition of ingredients as shown in Example 1 was used for preparing the tablet cores by direct compression. All the ingredients were blended and then compressed into tablet cores. The process was complicated due to poor flow properties of the material. Furthermore, massive sticking of the material to the equipment, mainly on punches occurred already after production a few tablet cores. The manufacturing process had to be stopped without the production being completed.

Another attempt was made and the agomelatine co-crystals with citric acid were first pre-blended with one third (by weight) of the total amount of the silicified microcrystalline cellulose and a part of the colloidal anhydrous silica (app. 85% of total amount by weight) and homogenized by blending in Turbula mixer at 32 ± 2 RPM for 10 min. Other the rest of the excipients were added to the pre-blend and homogenised by blending in Turbula mixer at 32 ± 2 RPM for 10 min. The resulting mixture was compressed into tablet cores and film-coated.

### Results:

The common method of direct compression was not suitable for preparation of the tablets comprising agomelatine co-crystals. The pre-blending step of agomelatine co-crystals and glidant solved the technological problem with sticking.

### Example 4 - Influence of particle size distribution

The influence of particle size of the agomelatine in the form of co-crystals on the sticking problem as well as on the dissolution profiles of the resulting film-coated tablets was tested.

The particle size distribution of agomelatine in the form of co-crystals in these three batches was as follows (measured by scanning electron microscope (SEM) method):

| | |
|---|---|
| Batch No. 500812/A | 90 % particles below 12 µm |
| Batch No. 500812/C | 90 % particles below 130 µm |
| Batch No. 500812/D | 90 % particles below 60 µm |

### a/ manufacturing process according to the invention

Three batches of film-coated tablets were prepared by identical manufacturing process as in the Example 1. For each batch were used agomelatine co-crystals with different particle size distribution. The qualitative and quantitative composition was identical as well.

### b/ manufacturing process without pre-blending step

Three batches were produced by the process described in the Example 3a/. For each batch were used agomelatine co-crystals with different particle size distribution. The qualitative and quantitative composition was identical as well.

### Results:

No influence of the particle size of the agomelatine in the form of co-crystals on the sticking problem was observed.

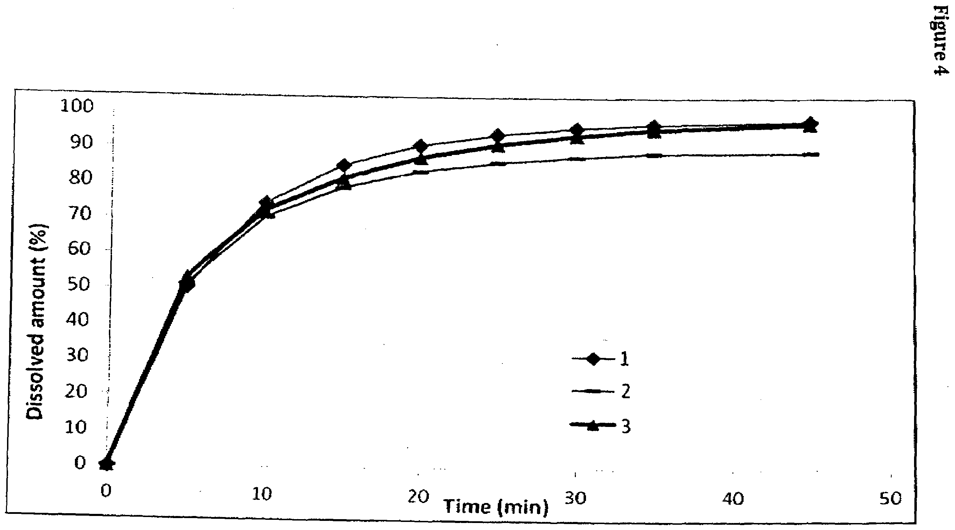

In the case a/, all three batches were produced without any technological problems and the dissolution profiles were measured at pH 2 (0.01 M HCl); 900ml media; paddles; 50 RPM; after 45 minutes 150 RPM (results shown in Figure 4). Neither the manufacturing process nor the dissolution profiles were affected by the particle size distribution of the agomelatine in the form of co-crystals. No significant change of the dissolution profile is found within the tested particle size range and thus the particle size range is optimal.

In the case b/, significant problems with sticking of the mass to the equipment occurred during the manufacture of all three batches.

The particle size distribution of the agomelatine co-crystals had no effect on the sticking problem.

### Example 5 - Influence of tablet hardness on dissolution

Three batches with different tablet hardness (according to method described in European Pharmacopeia) of the same composition and manufacturing process as described in the Example 1 were tested (66 N, 96 N and 146 N) to find an influence of this parameter on the problem with sticking within the manufacturing process as well as on the dissolution profiles.

### Results:

No effect of the compressing force on the problem with sticking was observed. No significant influence on dissolution profile was found. Slightly slower dissolution has been found for the tablets of higher hardness about 146 N.

### Example 6 - Composition and process according to the invention

**Table 7**

| **Ingredient** | **Function** | **Amount in tablet** | |
|---|---|---|---|
| | | mg | % |
| **Tablet core or capsule fill** | | | |
| Agomelatine* | active ingredient | 25.00 | 18.7 |
| Citric acid* | co-crystal former | 19.74 | 14.7 |
| Microcrystalline cellulose | filler, glidant | 60.77 | 45.3 |
| Polyvinylpyrrolidone 30 | binder | 4.06 | 3.0 |
| Colloidal silica | glidant | 6.50 | 4.9 |
| Crospovidone | disintegrant | 7.80 | 5.8 |
| Sodium stearyl fumarate | lubricant | 3.70 | 2.8 |
| Magnesium stearate | lubricant | 0.50 | 0.4 |
| Stearic acid | lubricant | 2.00 | 1.5 |

| **Optionally: film-coating** | | | |
|---|---|---|---|
| Methocel E5 | film-forming agent | 2.39 | 1.8 |
| Macrogol 6000 | plasticizer | 0.57 | 0.4 |
| Titanium dioxide | colourant | 0.30 | 0.2 |
| Talc | colourant | 0.61 | 0.5 |
| Ferric oxide yellow | colourant | 0.12 | 0.1 |
| **Total tablet mass** | | **134.00** | **100**.**0** |

| | | | |
|---|---|---|---|
| *present together as a parts of co-crystal in the drug product | | | |

### Manufacturing Process:

The same composition as in the Example 1 was prepared, however, the silicified microcrystalline cellulose was replaced by microcrystalline cellulose. The manufacturing process was similar to that used in the Example 1:

### A/ PRE-BLENDING

Agomelatine co-crystal with citric acid and a part of the colloidal anhydrous silica (app. 85% of total amount by weight) were sieved through 0.8 ± 0.2 mm sieve and homogenized by blending in Turbula mixer at 32 ± 2 RPM for 10 min.

### B/ HOMOGENIZATION PRIOR TO DRY COMPACTION

Microcrystalline cellulose, povidone 30, sodium stearyl fumarate and a part of crospovidon (app. 50 % of total amount by weight) were sieved through 0.8 ± 0.2 mm sieve and added to the pre-blend formed in the step A/ and homogenized again in Turbula mixer at 32 ± 2 RPM for 15 min.

### C/ DRY GRANULATION AND SIEVING OF GRANULES

The homogenized mixture was granulated by roller compactor under pressure 40 - 65 bar and the granulate was sieved through 1.0 ± 0.2 mm sieve.

### D/ PREPARATION OF MIXTURE FOR TABLETING OR FILLING INTO CAPSULES

The rest of the colloidal anhydrous silica and the rest of crospovidon were sieved through 0.8 ± 0.2 mm sieve and added to the granulate of the step C/, and homogenized in Turbula mixer at 32 ± 2 RPM for 20 min. After that stearic acid and magnesium stearate were sieved through the same sieve and added into mixture and homogenized in Turbula mixer at 32 ± 2 RPM for 5 min.

### E/ COMPRESSION

The mixture ready for compression from the previous step was compressed using a rotary tableting machine at commonly used conditions. This mixture would be suitable for filling into capsules as well, if required.

The resulting tablet cores were oblong, biconvex, size of approximately 9 x 4.5 mm (length x width) and average mass 130 mg ± 5 %.

### F/ COATING

Coating suspension was prepared using Methocel E5, Macrogol 6000, Titanium dioxide, Talc, Ferric oxide yellow and purified water. Cores were film coated using coating suspension in a coater machine. The film-coated tablets were yellow oblong biconvex film-coated tablets, size of approximately 9 x 4.5 mm (length x width) and average mass 134 mg ± 5 %.

### Results:

The manufacturing process was easy to handle, no problems with sticking of the material to the equipment was observed.

## Claims

1. A pharmaceutical formulation of agomelatine for oral use, comprising
- agomelatine in the form of agomelatine co-crystals with organic acids and
- pharmaceutically acceptable excipients,
characterised that the formulation is obtained in that
- the agomelatine co-crystals are used in the form of pre-blend of the agomelatine co-crystals with a glidant,
- the ratio of the agomelatine co-crystals to the glidant in the pre-blend is lower than or equal to 30 : 1, and
- the free-water content in the used pharmaceutically acceptable excipients is lower than or equal to 7% w/w.

2. The pharmaceutical formulation according to claim 1, wherein the ratio of the agomelatine co-crystals to the glidant in the pre-blend is lower than 15 : 1.

3. The pharmaceutical formulation according to claims 1 or 2, wherein the ratio of the agomelatine co-crystals to the glidant in the pre-blend is between 5 : 1 and 10 : 1 including the limit values.

4. The pharmaceutical formulation according to any of claims 1-3, wherein agomelatine is in the form of agomelatine co-crystal with citric acid.

5. The pharmaceutical formulation according to claim 4, wherein agomelatine is in the form of agomelatine co-crystal with citric acid in the molar ratio of agomelatine to citric acid being 1:1.

6. The pharmaceutical formulation according to any one of claims 1-5, wherein the particle size distribution of the agomelatine co-crystals is **characterized by** D90 value between 10 and 200 µm including the limit values.

7. The pharmaceutical formulation according to any one of preceding claims, comprising (by weight)
▪ 10-50% agomelatine co-crystals
▪ 2-10% one or more glidants, out of which 1.7-10% are in the form of pre-blend with agomelatine co-crystals
▪ 30-80% one or more fillers
▪ 1-5% one or more binders
▪ 2-10% one or more disintegrants
▪ 0.5-7% lubricants
▪ optionally up to 5% film forming agents
▪ optionally up to 2% plasticizers
▪ optionally up to 1% pigments.

8. The pharmaceutical formulation according to any one of preceding claims, comprising (by weight)
▪ 30-40% agomelatine co-crystals
▪ 5-7% one or more glidants, out of which 4.7-7% are in the form of pre-blend with agomelatine co-crystals
▪ 35-55% one or more fillers
▪ 3-5% one or more binders
▪ 6-10% one or more disintegrants
▪ 0.5-7% lubricants
▪ optionally up to 5% film forming agents
▪ optionally up to 2% plasticizers
▪ optionally up to 1% pigments.

9. The pharmaceutical formulation according to any one of preceding claims, wherein the pharmaceutical formulation is in the form of tablets, capsules or granulates.

10. The pharmaceutical formulation according to any one of preceding claims, **characterized by** the composition shown in the following table
| **Ingredient** | **Function** | **Amount in tablets** | |
|---|---|---|---|
| | | mg | % |
| Agomelatine | active ingredient | 25.00 | 18.7 |
| Citric acid | co-crystal former | 19.74 | 14.7 |
| Silicified microcrystalline cellulose | filler, glidant | 60.77 | 45.3 |
| Polyvinylpyrrolidone 30 | binder | 4.06 | 3.0 |
| Colloidal silica | glidant | 6.50 | 4.9 |
| Crospovidone | disintegrant | 7.80 | 5.8 |
| Sodium stearyl fumarate | lubricant | 3.70 | 2.8 |
| Magnesium stearate | lubricant | 0.50 | 0.4 |
| Stearic acid | lubricant | 2.00 | 1.5 |
wherein the agomelatine is in the form of its co-crystals with the citric acid and wherein one third of the silicified microcrystalline cellulose and 85% by weight of the total amount of colloidal silica are used in the form of pre-blend with the agomelatine co-crystals.

11. The pharmaceutical formulation according to any one of claims 7-10, wherein the pharmaceutical formulation is in the form of granulate and is filled into capsules or into sachets.

12. The pharmaceutical formulation according to any one of claims 7-10, wherein the pharmaceutical formulation is in the form of tablets.

13. The pharmaceutical formulation according to claim 12, wherein the tablets are further film-coated.

14. A manufacturing process of a pharmaceutical formulation for oral use comprising agomelatine, **characterized in that** the manufacturing process is a dry process and it comprises pre-blending step of agomelatine in the form of agomelatine co-crystals with organic acids and at least one glidant, wherein the ratio of the agomelatine co-crystals to the glidant in the pre-blend is lower than or equal to 30 : 1.

15. The manufacturing process according to claim 14, wherein the ratio of the agomelatine co-crystals to the glidant in the pre-blend is lower than or equal to 15 : 1.

16. The manufacturing process according to claims 14 or 15, wherein the ratio of the agomelatine co-crystals to the glidant in the pre-blend is between 5 : 1 and 10 : 1 including the limit values.

17. The manufacturing process according to any one of claims 14-16, wherein the pre-blend of the agomelatine co-crystals and the glidant is further processed by the process of direct compression into tablets.

18. The manufacturing processes according to claim 17, wherein the tablets are further film-coated.

19. The manufacturing process according to any one of claims 14-16, wherein the pre-blend of the agomelatine co-crystals and the glidant is further processed by the process of dry granulation into a granulate.

20. The manufacturing process according to claim 19, wherein the dry granulation is roller compaction.

21. The manufacturing process according to claim 19 or 20, wherein the granulate is filled into capsules.

22. The manufacturing process according to claim 19 or 20, wherein the granulate is filled into sachets.

23. The manufacturing process according to claim 19 or 20, wherein the granulate is compressed into tablets.

24. The manufacturing process according to claim 23, wherein the tablets are further film-coated.

25. The manufacturing process according to any one of claims 14-24, wherein agomelatine is in the form of agomelatine co-crystal with citric acid.

26. The manufacturing process according to claim 25, wherein agomelatine is in the form of agomelatine co-crystal with citric acid in the molar ratio of agomelatine to citric acid being 1:1.

## Patentansprüche

1. Pharmazeutische Formulierung von Agomelatin zur oralen Verwendung, umfassend
- Agomelatin in Form von Agomelatin-Kokristallen mit organischen Säuren, und
- pharmazeutisch annehmbare Hilfsstoffe,
**dadurch gekennzeichnet, dass**
- die Agomelatin-Kokristalle in Form einer Vormischung der Agomelatin-Kokristalle mit einem Fließregulierungsmittel verwendet werden,
- das Verhältnis der Agomelatin-Kokristalle zu dem Fließregulierungsmittel in der Vormischung niedriger als, oder gleich, 30:1 ist und
- der Gehalt an freiem Wasser in den verwendeten pharmazeutisch annehmbaren Hilfsstoffen niedriger als, oder gleich, 7 % w/w ist.

2. Pharmazeutische Formulierung nach Anspruch 1, wobei das Verhältnis der Agomelatin-Kokristalle zu dem Fließregulierungsmittel in der Vormischung niedriger als 15:1 ist.

3. Pharmazeutische Formulierung nach Anspruch 1 oder 2, wobei das Verhältnis der Agomelatin-Kokristalle zu dem Fließregulierungsmittel in der Vormischung zwischen 5:1 und 10:1 beträgt, einschließlich der Grenzwerte.

4. Pharmazeutische Formulierung nach einem der Ansprüche 1-3, worin Agomelatin in Form von Agomelatin-Kokristall mit Citronensäure vorliegt.

5. Pharmazeutische Formulierung nach Anspruch 4, worin Agomelatin in Form von Agomelatin-Kokristall mit Citronensäure vorliegt, wobei das Molverhältnis von Agomelatin zu Citronensäure 1:1 beträgt.

6. Pharmazeutische Formulierung nach einem der Ansprüche 1-5, wobei die Partikelgrößenverteilung der Agomelatin-Kokristalle durch einen D90-Wert zwischen 10 und 200 µm, einschließlich der Grenzwerte, gekennzeichnet ist.

7. Pharmazeutische Formulierung nach einem der vorhergehenden Ansprüche, umfassend (nach Gewicht)
- 10-50 % Agomelatin-Kokristalle
- 2-10 % ein oder mehrere Fließregulierungsmittel, von denen 1,7 bis 10 % in Form einer Vormischung mit Agomelatin-Kokristallen vorliegen
- 30-80 % ein oder mehrere Füllmittel
- 1-5 % ein oder mehrere Bindemittel
- 2-10 % ein oder mehrere Sprengmittel
- 0,5-7 % Gleitmittel
- gegebenenfalls bis zu 5 % Filmbildner
- gegebenenfalls bis zu 2 % Weichmacher
- gegebenenfalls bis zu 1 % Pigmente.

8. Pharmazeutische Formulierung nach einem der vorhergehenden Ansprüche, umfassend (nach Gewicht)
- 30-40 % Agomelatin-Kokristalle
- 5-7 % ein oder mehrere Fließregulierungsmittel, davon liegen 4,7-7 % in Form einer Vormischung mit Agomelatin-Kokristallen vor
- 35-55 % ein oder mehrere Füllmittel
- 3-5 % ein oder mehrere Bindemittel
- 6-10 % ein oder mehrere Sprengmittel
- 0,5-7 % Gleitmittel
- gegebenenfalls bis zu 5 % Filmbildner
- gegebenenfalls bis zu 2 % Weichmacher
- gegebenenfalls bis zu 1 % Pigmente.

9. Pharmazeutische Formulierung nach einem der vorhergehenden Ansprüche, wobei die pharmazeutische Formulierung in Form von Tabletten, Kapseln oder Granulaten vorliegt.

10. Pharmazeutische Formulierung nach einem der vorhergehenden Ansprüche, **gekennzeichnet durch** die in der folgenden Tabelle dargestellte Zusammensetzung
| Ingredienz | Funktion | Menge in Tablette | |
|---|---|---|---|
| | | mg | % |
| Agomelatin | Wirkstoff | 25,00 | 18,7 |
| Citronensäure | Kokristallbildner | 19,74 | 14,7 |
| Verkieselte mikrokristalline Cellulose | Füllmittel, Fließregulierungsmittel | 60,77 | 45,3 |
| Polyvinylpyrrolidon 30 | Bindemittel | 4,06 | 3,0 |
| Kolloidales Siliciumdioxid | Fließregulierungsmittel | 6,50 | 4, 9 |
| Crospovidon | Sprengmittel | 7,80 | 5,8 |
| Natriumstearylfumarat | Gleitmittel | 3,70 | 2,8 |
| Magnesiumstearat | Gleitmittel | 0,50 | 0,4 |
| Stearinsäure | Gleitmittel | 2,00 | 1,5 |
wobei das Agomelatin in Form seiner Kokristalle mit der Citronensäure vorliegt und wobei ein Drittel der verkieselten mikrokristallinen Cellulose und 85 Gew.-% der Gesamtmenge an kolloidalem Siliciumdioxid in Form einer Vormischung mit den Agomelatin-Kokristallen verwendet werden.

11. Pharmazeutische Formulierung nach einem der Ansprüche 7-10, wobei die pharmazeutische Formulierung in Form von Granulat vorliegt und in Kapseln oder in Sachets abgefüllt ist.

12. Pharmazeutische Formulierung nach einem der Ansprüche 7-10, wobei die pharmazeutische Formulierung in Form von Tabletten vorliegt.

13. Pharmazeutische Formulierung nach Anspruch 12, wobei die Tabletten des Weiteren mit einem Film überzogen sind.

14. Verfahren zur Herstellung einer Agomelatin enthaltenden pharmazeutischen Formulierung zur oralen Verwendung, **dadurch gekennzeichnet, dass** das Herstellungsverfahren ein Trockenverfahren ist und einen Schritt des Vormischens von Agomelatin in Form von Agomelatin-Kokristallen mit organischen Säuren und mindestens einem Fließregulierungsmittel umfasst, wobei das Verhältnis der Agomelatin-Kokristalle zu dem Fließregulierungsmittel in der Vormischung niedriger als, oder gleich, 30:1 ist.

15. Herstellungsverfahren nach Anspruch 14, wobei das Verhältnis der Agomelatin-Kokristalle zu dem Fließregulierungsmittel in der Vormischung niedriger als, oder gleich, 15:1 ist.

16. Herstellungsverfahren nach Anspruch 14 oder 15, wobei das Verhältnis der Agomelatin-Kokristalle zu dem Fließregulierungsmittel in der Vormischung zwischen 5:1 und 10:1, einschließlich der Grenzwerte, beträgt.

17. Herstellungsverfahren nach einem der Ansprüche 14-16, wobei die Vormischung der Agomelatin-Kokristalle und des Fließregulierungsmittels durch das Verfahren der Direkttablettierung zu Tabletten weiterverarbeitet wird.

18. Herstellungsverfahren nach Anspruch 17, wobei die Tabletten des Weiteren mit einem Film überzogen werden.

19. Herstellungsverfahren nach einem der Ansprüche 14-16, wobei die Vormischung der Agomelatin-Kokristalle und des Fließregulierungsmittels weiterhin durch das Verfahren der Trockengranulierung zu einem Granulat verarbeitet wird.

20. Herstellungsverfahren nach Anspruch 19, wobei die Trockengranulierung eine Walzenkompaktierung ist.

21. Herstellungsverfahren nach Anspruch 19 oder 20, wobei das Granulat in Kapseln gefüllt wird.

22. Herstellungsverfahren nach Anspruch 19 oder 20, wobei das Granulat in Sachets gefüllt wird.

23. Herstellungsverfahren nach Anspruch 19 oder 20, wobei das Granulat zu Tabletten verpresst wird.

24. Herstellungsverfahren nach Anspruch 23, wobei die Tabletten weiterhin mit einem Film überzogen werden.

25. Herstellungsverfahren nach einem der Ansprüche 14-24, wobei Agomelatin in der Form von Agomelatin-Kokristall mit Citronensäure vorliegt.

26. Herstellungsverfahren nach Anspruch 25, wobei Agomelatin in der Form von Agomelatin-Kokristall mit Citronensäure vorliegt, wobei das Molverhältnis von Agomelatin zu Citronensäure 1:1 beträgt.

## Revendications

1. Une formulation pharmaceutique d'agomélatine à usage oral, comprenant
- de l'agomélatine sous la forme de co-cristaux d'agomélatine avec des acides organiques et
- des excipients pharmaceutiquement acceptables, **caractérisée en ce que** la formulation est obtenue dans les conditions où :
- les co-cristaux d'agomélatine sont utilisés sous la forme de pré-mélange de co-cristaux d'agomélatine avec un agent de glissement,
- le rapport co-cristaux d'agomélatine/agent de glissement dans le pré-mélange est inférieur ou égal à 30/1, et
- la teneur en eau libre dans les excipients pharmaceutiquement acceptables utilisés est inférieure à ou égal à 7% p/p.

2. La formulation pharmaceutique selon la revendication 1, dans laquelle le rapport co-cristaux d'agomélatine/agent de glissement dans le pré-mélange est inférieur à 15/1.

3. La formulation pharmaceutique selon la revendication 1 ou 2, dans laquelle le rapport co-cristaux d'agomélatine/agent de glissement dans le pré-mélange est compris entre 5/1 et 10/1 incluant les valeurs limites.

4. La formulation pharmaceutique selon l'une quelconque des revendications 1 à 3, dans laquelle l'agomélatine est sous la forme d'un co-cristal d'agomélatine avec de l'acide citrique.

5. La formulation pharmaceutique selon la revendication 4, dans laquelle l'agomélatine se présente sous la forme d'un co-cristal d'agomélatine avec de l'acide citrique, le rapport molaire agomélatine/acide citrique étant de 1/1.

6. La formulation pharmaceutique selon l'une quelconque des revendications 1 à 5, dans laquelle la distribution granulométrique des co-cristaux d'agomélatine est **caractérisée par** une valeur de D90 comprise entre 10 et 200 µm incluant les valeurs limites.

7. La formulation pharmaceutique selon l'une quelconque des revendications précédentes, comprenant (en poids)
▪ 10 à 50% de co-cristaux d'agomélatine
▪ 2 à 10% d'un ou plusieurs agents de glissement, parmi lesquels 1,7 à 10% sont sous forme de pré-mélange avec des co-cristaux d'agomélatine
▪ 30 à 80% d'une ou plusieurs charges
▪ 1 à 5% d'un ou plusieurs liants
▪ 2 à 0% d'un ou plusieurs agents délitant
▪ 0,5 à 7% de lubrifiants
▪ éventuellement jusqu'à 5% d'agents filmogènes
▪ éventuellement jusqu'à 2% d'agents plastifiants
▪ éventuellement jusqu'à 1% de pigments.

8. La formulation pharmaceutique selon l'une quelconque des revendications précédentes, comprenant (en poids)
▪ 30 à 40% de co-cristaux d'agomélatine
▪ 5 à 7% d'un ou plusieurs agents de glissement, parmi lesquels 4,7 à 7% sont sous forme de pré-mélange avec des co-cristaux d'agomélatine
▪ 35 à 55% d'une ou plusieurs charges
▪3 à 5% d'un ou plusieurs liants
▪ 6 à 10% d'un ou plusieurs agents délitant
▪ 0,5 à 7% d'agents lubrifiants
▪ éventuellement jusqu'à 5% d'agents filmogènes
▪ éventuellement jusqu'à 2% de plastifiants
▪ éventuellement jusqu'à 1% de pigments.

9. La formulation pharmaceutique selon l'une quelconque des revendications précédentes, dans laquelle la formulation pharmaceutique se présente sous la forme de comprimés, de capsules ou de granulés.

10. La formulation pharmaceutique selon l'une quelconque des revendications précédentes, **caractérisée par** la composition présentée dans le tableau suivant
| INGREDIENT | FONCTION | Quantité en comprimés | |
|---|---|---|---|
| | | mg | % |
| Agomélatine | principe actif | 25,00 | 18,7 |
| Acide citrique | formateur de co-cristaux | 19,74 | 14,7 |
| Cellulose microcristalline silicifiée | charge, agent de glissement | 60,77 | 45,3 |
| Polyvinylpyrrolidone 30 | liant | 4,06 | 3,0 |
| Silice colloïdale | Agent de glissement | 6,50 | 4,9 |
| Crospovidone | délitant | 7,80 | 5,8 |
| Fumarate de stéaryle de sodium | lubrifiant | 3,70 | 2,8 |
| Stéarate de magnésium | lubrifiant | 0,50 | 0,4 |
| Acide stéarique | lubrifiant | 2,00 | 1,5 |
dans laquelle l'agomélatine se présente sous la forme de ses co-cristaux avec l'acide citrique et dans lequel un tiers de la cellulose microcristalline silicifiée et 85% en poids de la quantité totale de silice colloïdale sont utilisés sous forme de pré-mélange avec les co-cristaux d'agomélatine.

11. La formulation pharmaceutique selon l'une quelconque des revendications 7 à 10, dans laquelle la formulation pharmaceutique est sous forme de granulés et est contenue dans des capsules ou des sachets.

12. La formulation pharmaceutique selon l'une quelconque des revendications 7 à 10, dans laquelle la formulation pharmaceutique se présente sous la forme de comprimés.

13. La formulation pharmaceutique selon la revendication 12, dans laquelle les comprimés sont en outre enrobés de film.

14. Un procédé de fabrication d'une formulation pharmaceutique à usage oral comprenant de l'agomelatine, **caractérisé en ce que** le procédé de fabrication est un procédé à sec et comprend une étape de pré-mélange de l'agomélatine sous forme de co-cristaux d'agomélatine avec des acides organiques et au moins un agent de glissement, le rapport co-cristaux d'agomélatine/agent de glissement dans le pré-mélange étant inférieur ou égal à 30/1.

15. Le procédé de fabrication selon la revendication 14, dans lequel le rapport co-cristaux d'agomélatine/agent de glissement dans le pré-mélange est inférieur ou égal à 15/1.

16. Le procédé de fabrication selon la revendication 14 ou 15, dans lequel le rapport des co-cristaux d'agomélatine/agent de glissement dans le pré-mélange est compris entre 5/1 et 10/1 incluant les valeurs limites.

17. Le procédé de fabrication selon l'une quelconque des revendications 14 à 16, dans lequel le pré-mélange co-cristaux d'agomélatine et d'agent de glissement est en outre soumis à un procédé de compression directe en comprimés.

18. Le procédé de fabrication selon la revendication 17, dans lequel les comprimés sont en outre revêtus de film.

19. Le procédé de fabrication selon l'une quelconque des revendications 14 à 16, dans lequel le pré-mélange co-cristaux d'agomélatine et d'agent de glissement est en outre soumis à un procédé de granulation sèche pour former un granulé.

20. Le procédé de fabrication selon la revendication 19, dans lequel la granulation à sec est un compactage par rouleaux.

21. Le procédé de fabrication selon la revendication 19 ou 20, dans lequel le granulé est introduit dans des capsules.

22. Le procédé de fabrication selon la revendication 19 ou 20, dans lequel le granulé est introduit dans des sachets.

23. Le procédé de fabrication selon la revendication 19 ou 20, dans lequel le granulé est comprimé en comprimés.

24. Le procédé de fabrication selon la revendication 23, dans lequel les comprimés sont en outre revêtus de film.

25. Le procédé de fabrication selon l'une quelconque des revendications 14 à 24, dans lequel l'agomélatine se présente sous la forme d'un co-cristal d'agomélatine avec de l'acide citrique.

26. La formulation pharmaceutique selon la revendication 25, dans laquelle l'agomélatine se présente sous la forme d'un co-cristal d'agomélatine avec de l'acide citrique dans le rapport molaire agomélatine/acide citrique étant de 1/1.
